# EUROPÄISCHE PATENTSCHRIFT

(11) **EP 1 482 998 B1**
(45) Veröffentlichungstag und Bekanntmachung des Hinweises auf die Patenterteilung: **10.12.2008**
(21) Anmeldenummer: 03700795.2
(22) Anmeldetag: 07.02.2003
(51) Int. Cl.: A61M 1/06

(54) **BRUSTHAUBE**
BREAST CUP
CLOCHE DE TIRE-LAIT

(30) Priorität: 07.02.2002 WO PCT/CH02/00073
(43) Veröffentlichungstag der Anmeldung: 08.12.2004
(73) Patentinhaber: Medela Holding AG, 6340 Baar (CH)
(72) Erfinder: LARSSON, Michael, CH-6300 Zug (CH)
(74) Vertreter: Clerc, Natalia
(86) Internationale Anmeldenummer: PCT/CH2003/000094
(87) Internationale Veröffentlichungsnummer: WO 2003/066133

(56) Entgegenhaltungen:
- EP-A- 0 875 257
- WO-A-00/41618
- WO-A-00/47247
- WO-A-01/54488
- DE-A- 3 429 057
- US-A- 5 827 191

## Beschreibung

### Technisches Gebiet

Die vorliegende Erfindung betrifft eine Brusthaube, insbesondere für Brustpumpen, und ein Verfahren zur Detektion von Veränderungen in einer Mutterbrust beim Absaugen von Muttermilch aus der Mutterbrust.

### Stand der Technik

Brusthauben für Absaugpumpen bzw. -geräte zum Absaugen von Muttermilch sind in den verschiedensten Ausführungen weit verbreitet und stehen weltweit im Einsatz.

Das Stillen eines Säuglings ist nicht immer einfach. Gründe hierfür können sowohl bei der Mutter wie auch beim Säugling liegen. Auch das Absaugen der Muttermilch mittels Absaugpumpen oder -geräten, auch Brustpumpe genannt, kann einzelnen Müttern Schwierigkeiten bereiten.

Bei der Entwicklung von Absaugpumpen und deren Betriebsbedingungen wird deshalb heute mehr und mehr darauf geachtet, besser zu verstehen, was während dem Absaugen von Muttermilch im Innern der Mutterbrust vor sich geht.

Während dem Einsatz der Brustpumpe lassen sich aber nur schlecht Messungen an der Mutterbrust durchführen. Gerade solche Messungen würden jedoch Aufschluss über Vorgänge während der Stimulationsphase und dem eigentlichen Absaugen geben.

### Darstellung der Erfindung

Es ist deshalb Aufgabe der vorliegenden Erfindung, eine Vorrichtung sowie ein Verfahren zu schaffen, welche Messungen über Vorgänge im Innern der Brust problemlos auch während dem Absaugen von Milch zulassen.

Diese Aufgabe, lösen eine Brusthaube sowie ein Verfahren mit den Merkmalen der Patentansprüche 1 beziehungsweise 12.

Die überraschend einfache erfindungsgemässe Lösung verwendet eine Brusthaube, welche gemäss Anspruch 1 ausgebildet ist. Erfindungsgemäss wird somit eine Brusthaube verwendet, welche mit Sensoren versehen ist. Als Sensoren eignen sich alle Sensoren zur Detektion von Veränderungen in der Mutterbrust, insbesondere Elektroden, optische Sensoren, akustische oder thermische Sensoren.

Entsprechend bieten sich verschiedene Messverfahren an, wie z.B. Röntgenstrahlen, Ultraschall, rein elektronische Messströme, welche Wiederstand und Impedanz zwischen zwei im Abstand voneinander liegenden Stellen der Brust aufnehmen usw. Zur Messung der elektronische Signale werden Elektroden auf die Brusthaut aufgesetzt, zur Messung der Röntgenstrahlen optische Sensoren, von Ultraschall akustische Sensoren.

Die erfindungsgemäss Konstruktion der Brusthaube erlaubt Untersuchungen während dem Absaugen von Muttermilch. Durch das erfindungsgemässe Verfahren und die erfindungsgemässe Vorrichtung lassen sich insbesondere Erkenntnisse über den sogenannten Milcheinschuss gewinnen.

Weitere vorteilhafte Ausführungsformen und Varianten des Verfahren gehen aus den abhängigen Patentansprüchen hervor.

### Kurze Beschreibung der Zeichnung

Ausführungsbeispiele einer erfindungsgemässen Brusthaube werden nachstehend anhand der Zeichnung noch etwas näher erläutert. Es zeigen:
- Figur 1: eine perspektivische, rein schematische Darstellung einer erfindungsgemässen Brusthaube und
- Figur 2: eine ähnliche Darstellung mit Draufsicht auf das offene Ende der Brusthaube nach Figur 1.

### Wege zur Ausführung der Erfindung

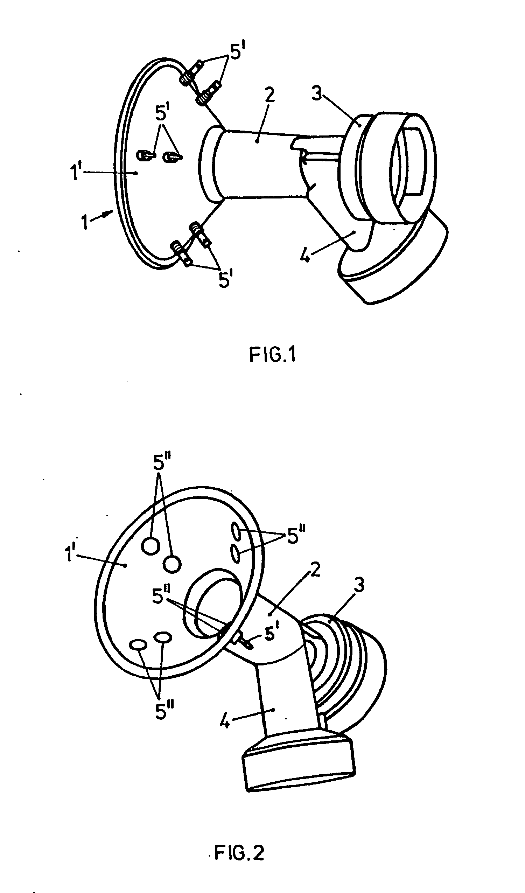

Figur 1 zeigt eine Brusthaube 1 von kegelstumpfförmiger Gestalt, welche über einen rohrförmigen Ansatz 2 zu Anschlüssen 3 für eine Brustpumpe (nicht dargestellt) und 4 für eine Milch-Auffangbehälter (nicht dargestellt) führt.

Die Haube 1 mit den Teilen 2 - 4 selbst, aus z.B. durchsichtigem Kunststoff, ist von konventioneller Bauart. Der Haubentrichter 1' ist mit einer Anzahl an vorbestimmten Stellen angeordneten Elektroden 5 versehen. Auf der Innenseite der Trichterwand 1' weisen die Elektroden Kontaktflächen 5" und auf der Aussenseite der Trichterwand Anschlusskontakt 5' auf.

Die Kontaktflächen 5" liegen bei auf eine Brust aufgesetzter Haube 1 auf der Brusthaube an, während über die Anschlusskontakte 5' (als Teile der durch die Trichterwand führenden Elektroden) eine gewünschte Spannung angelegt werden kann (jeweils ein Eingang und Ausgang).

Dieser Haubenaufbau erlaubt Messungen über die Elektroden auch während dem Absaugen von Muttermilch. Das entsprechende Auswertungsgerät der Messungen kann in der Brustpumpe integriert sein oder ein getrenntes Gerät sein.

Anstelle oder zusätzlich zu den Elektroden 5 können auch andere Arten von Sensoren in der Brusthaube angeordnet sein, beispielsweise akustische Sensoren, optische Sensoren oder thermische Sensoren. Je nach Art des Sensors muss er nicht zwingend eine Kontaktfläche 5" aufweisen. Beispielsweise bei optischen Sensoren genügt es, wenn sie genügend nahe an die Brustoberfläche gebracht werden. Bei Verwendung von optischen Sensoren empfiehlt es sich, mindestens den Bereich der Brusthaube. welche den Sensor benachbart umgibt, für die zur Messung verwendeten optische Wellenlänge durchsichtig zu gestalten oder zumindest keine Reflexionen an der Brusthaubeninnenseite zuzulassen.

Je nach Art der verwendeten Sensoren muss die Brusthaube beziehungsweise ihr Grundkörper nicht zwingend aus einem elektrisch nichtleitenden Material bestehen. Im Falle der Verwendung von Elektroden als Sensoren kann es auch genügen, lediglich die Bereiche der Brusthaube, welche den Elektroden benachbart sind, elektrisch nichtleitend zu gestalten.

Wie in den Figuren erkennbar ist, ragt die Brustwarze der Mutterbrust in die Trichteröffnung hinein. Die Sensoren sind somit beabstandet zur Brustwarze angeordnet, so dass diese während dem Absaugen nicht gestört wird.

## Patentansprüche

1. Brusthaube einer Brustpumpe zur Anlage auf einer Mutterbrust, **dadurch gekennzeichnet, dass** in der Brusthaube Sensoren angeordnet sind, welche geeignet sind, während dem Absaugen von Muttermilch Vorgänge im Innern der Mutterbrust zu detektieren.

2. Brusthaube nach Anspruch 1, mit einem trichterförmigen Grundkörper aus elektrisch nichtleitendem Material, welcher dazu vorgesehen ist, mit Berfihrungskontakt auf eine Brust aufgesetzt zu werden, wobei die Sensoren Elektroden sind, welche in der trichterförmigen Wand des Grundkörpers an vorbestimmten Stellen eingebaut sind und welche auf der Trichterinnenseite Kontaktflächen für die Brusthaut aufweisen, durch die Trichterwand führen und auf der Trichteraussenseite elektrische Anschlusskontakte aufweisen.

3. Brusthaube nach Anspruch 2, **dadurch gekennzeichnet, dass** der Grundkörper kegelstumpfförmig ausgebildet ist und am im Durchmesser kleineren Ende eine rohrförmige verlängerung aufweist, welche zu Anschlüssen für einen Milchauffangbehälter und eine Absaugpumpe führt.

4. Brusthaube nach Anspruch 1, **dadurch gekennzeichnet, dass** die Sensoren beabstandet zur Brustwarze angeordnet sind.

5. Brusthaube nach einem der Ansprüche 1 oder 4, **dadurch gekennzeichnet, dass** die Brusthaube aus einem trichterförmigen Grundkörper besteht, welcher dazu vorgesehen ist, mit Berührungskontakt auf die Mutterbrust aufgesetzt zu werden und dass die Sensoren in der trichterförmigen Wand des Grundkörpers angeordnet sind.

6. Brusthaube nach Anspruch 5, **dadurch gekennzeichnet, dass** die Sensoren an vorbestimmten Stellen in die Wand des Grundkörpers eingebaut sind.

7. Brusthaube nach einem der Ansprüche 5 oder 6, **dadurch gekennzeichnet, dass** die Sensoren auf der der Mutterbrust zugewandten Innenseite des Grundkörpers Kontaktflächen für die Brusthaut aufweise.

8. Brusthaube nach einem der Ansprüche 1, 4 bis 7, **dadurch gekennzeichnet, dass** die Sensoren Elektroden sind.

9. Brusthaube nach den Ansprüchen 5 und 8, **dadurch gekennzeichnet, dass** die Elektroden durch die Trichterwand führen und auf der Trichteraussenseite elektrische Anschlusskontakte aufweisen.

10. Brusthaube nach einem der Ansprüche 1, 4 bis 9, **dadurch gekennzeichnet, dass** sie optische Sensoren aufweist.

11. Brusthaube nach einem der Ansprüche 1, 4 bis 9, **dadurch gekennzeichnet, dass** sie akustische und/oder thermische Sensoren aufweist.

12. Verfahren zur Messung von Veränderungen in einer Mutterbrust beim Absaugen von Muttermilch aus der Mutterbrust, wobei eine Brustpumpe mit einer mit Sensoren versehenen Brusthaube verwendet wird, wobei die Brusthaube während des Absaugens auf die Mutterbrust aufgesetzt ist, so dass die Sensoren beabstandet zur Brustwarze sind, und wobei die Veränderungen gemessen werden, indem die Sensoren Vorgänge im Innern der Mutterbrust während dem Absaugen der Muttermilch detektieren.

13. Verfahren nach Anspruch 12, **dadurch gekennzeichnet, dass** die Sensoren diese Vorgänge elektrisch, optisch, mittels wärmemessung oder mittels Schall detektieren.

14. Verfahren nach Anspruch 12, **dadurch gekennzeichnet, dass** eine Impedanzmessung durchgeführt wird.

15. Verfahren nach Anspruch 12, **dadurch gekennzeichnet, dass** Ultraschall verwendet wird.

## Claims

1. The breast cup for a breast pump for placing on a mother's breast, **characterized in that** sensors are arranged in the breast cup, which sensors, during the expression of breast milk, are adapted for detecting processes in the inside of the mother's breast.

2. The breast cup according to claim 1 with a funnel-shaped main body made of electrically nonconducting material, which is intended to be placed with contact on a breast, wherein the sensors are electrodes which are incorporated in the funnel-shaped wall of the main body at predetermined locations, which electrodes, on the inside face of the funnel, have contact surfaces for the breast skin, lead through the funnel wall and, on the outside face of the funnel, have electrical connection contacts.

3. The breast cup as claimed in claim 2,
**characterized in that** the main body is of truncated cone shape and, at the end of smaller diameter, it has a tubular continuation which leads to attachments for a milk-collecting container and a suction pump.

4. The breast cup as claimed in claim 1,
**characterized in that** the sensors are arranged at a distance from the nipple.

5. The breast cup as claimed in either of claims 1 and 4, **characterized in that** the breast cup consists of a funnel-shaped main body which is intended to be placed with contact on the mother's breast and **in that** the sensors are arranged in the funnel-shaped wall of the main body.

6. The breast cup as claimed in claim 5, **characterized in that** the sensors are incorporated into the wall of the main body at predetermined locations.

7. The breast cup as claimed in either of claims 5 and 6, **characterized in that** the sensors, on the inside face of the main body facing the mother's breast, have contact surfaces for the breast skin.

8. The breast cup as claimed in one of claims 1, 4 through 7, **characterized in that** the sensors are electrodes.

9. The breast cup as claimed in claims 5 and 8, **characterized in that** the electrodes lead through the funnel wall and, on the outside face of the funnel, have electrical connection contacts.

10. The breast cup as claimed in one of claims 1, 4 through 9, **characterized in that** it has optical sensors.

11. The breast cup as claimed in one of claims 1, 4 through 9, **characterized in that** it has acoustic and/or thermal sensors.

12. A method for measuring changes in a mother's breast during expression of breast milk from the breast, in which method a breast pump having a breast cup provided with sensors is used, and in which the breast cup is placed on the mother's breast during expression of milk, so that the sensors are at a distance from the nipple, and the changes are measured by means of the sensors detecting processes in the inside of the mother's breast during expression of milk.

13. The method as claimed in claim 12, **characterized in that** the sensors detect these processes electrically, optically, by means of heat measurement, or by means of sound.

14. The method as claimed in claim 12, **characterized in that** an impedance measurement is carried out.

15. The method as claimed in claim 12, **characterized in that** ultrasound is used.

## Revendications

1. Cloche de tire-lait conçue pour l'application sur une mamelle, **caractérisée par le fait que** ladite cloche renferme des capteurs appropriés pour détecter, au cours de la succion de lait maternel, des phénomènes se produisant à l'intérieur de la mamelle.

2. Cloche selon la revendication 1, munie d'un corps de base infundibuliforme en un matériau électriquement non conducteur, prévu pour être appliqué sur une mamelle, les capteurs étant des électrodes qui sont intégrées dans la paroi infundibuliforme du corps de base, dans des zones prédéterminées ; comportent, sur la face intérieure de l'entonnoir, des surfaces de contact destinées à l'épiderme mammaire ; traversent la paroi de l'entonnoir ; et présentent des contacts électriques de raccordement sur la face extérieure de l'entonnoir.

3. Cloche selon la revendication 2, **caractérisée par le fait que** le corps de base est de réalisation tronconique et présente, à l'extrémité de diamètre réduit, un prolongement tubulaire menant à des raccords affectés à un réceptacle collecteur de lait et à une pompe d'aspiration.

4. Cloche selon la revendication 1, **caractérisée par le fait que** les capteurs sont placés à distance du mamelon.

5. Cloche selon l'une des revendications 1 ou 4, **caractérisée par le fait que** ladite cloche est constituée d'un corps de base infundibuliforme prévu pour être appliqué sur la mamelle ; et **par le fait que** les capteurs sont disposés dans la paroi de l'entonnoir dudit corps de base.

6. Cloche selon la revendication 5, **caractérisée par le fait que** les capteurs sont intégrés, dans des zones prédéterminées, dans la paroi du corps de base.

7. Cloche selon l'une des revendications 5 ou 6, **caractérisée par le fait que** les capteurs offrent, sur la face intérieure du corps de base tournée vers la mamelle, des surfaces de contact destinées à l'épiderme mammaire.

8. Cloche selon l'une des revendications 1, 4 à 7, **caractérisée par le fait que** les capteurs sont des électrodes.

9. Cloche selon les revendications 5 et 8, **caractérisée par le fait que** les électrodes traversent la paroi de l'entonnoir, et présentent des contacts électriques de raccordement sur la face extérieure dudit entonnoir.

10. Cloche selon l'une des revendications 1, 4 à 9, **caractérisée par le fait qu'**elle comporte des capteurs optiques.

11. Cloche selon l'une des revendications 1, 4 à 9, **caractérisée par le fait qu'**elle comporte des capteurs acoustiques et/ou thermiques.

12. Procédé de mesure de variations intervenant dans une mamelle au cours de la succion de lait maternel hors de ladite mamelle, sachant qu'il est fait usage d'un tire-lait muni d'une cloche équipée de capteurs ; sachant que ladite cloche est appliquée sur la mamelle au cours de la succion, de façon telle que lesdits capteurs se trouvent à distance du mamelon ; et sachant que lesdites variations sont mesurées du fait que lesdits capteurs détectent, au cours de la succion du lait maternel, des phénomènes se produisant à l'intérieur de ladite mamelle.

13. Procédé selon la revendication 12, **caractérisé par le fait que** les capteurs détectent ces phénomènes en mode électrique, optique, par mesure thermique ou en mode sonique.

14. Procédé selon la revendication 12, **caractérisé par** l'exécution d'une mesure d'impédance.

15. Procédé selon la revendication 12, **caractérisé par** l'utilisation d'ultrasons.
